# FASCICULE DE BREVET EUROPEEN

(11) **EP 0 767 167 B1**
(45) Date de publication et mention de la délivrance du brevet: **16.02.2000**
(21) Numéro de dépôt: 96402091.1
(22) Date de dépôt: 01.10.1996
(51) Int. Cl.: C07C 317/40, A61K 31/275

(54) **Nouveaux dérivés 2-cyano 3-hydroxy propénamides comprenant un radical trifluorométhylsulfonylphényle ou trifluorométhylsulfinylphényle, leur procédé de préparation, leur application à titre médicaments et les compositions pharmaceutiques les renfermant**
Eine Trifluormethylsulfonylphenyl- oder Trifluormethylsulfinylphenylgruppe enthaltende 2-Cyan-3-hydroxy-propenamidderivate, Verfahren zu ihrer Herstellung, ihre Anwendung als Arzneimittel und diese enthaltende pharmazeutische Zusammensetzungen
Trifluoromethylsulfonylphenyl or trifluoromethylsulfinylphenyl group containing 2-cyano-3-hydroxy-propenamide derivatives, process for their preparation, their use as medicaments and pharmaceutical compositions containing them

(30) Priorité: 02.10.1995 GB 9520092
(43) Date de publication de la demande: 09.04.1997
(73) Titulaire: HOECHST MARION ROUSSEL, 92800 Puteaux (FR)
(72) Inventeur: Kay, David Paul, Swindon SN3 5BZ (GB); Kuo, Elizabert Anne, Dorcan, Swindon, Wiltshire (GB); Williamson, Richard Alexander, Ickleford, Hitchin Herts SG5 3TH (GB)
(74) Mandataire: Vieillefosse, Jean-Claude

(56) Documents cités:
- EP-A- 0 484 223
- EP-A- 0 533 573
- EP-A- 0 551 230
- EP-A- 0 632 017
- WO-A-91/17748
- J. MED. CHEM., vol. 39, no. 23, 1996, pages 4608-4621, XP000196780 E.A. KUO ET AL:

## Description

La présente invention concerne des nouveaux 2-cyano-3-hydroxy-propénamides contenant un groupe trifluorométhylsulfonylphényle ou trifluorométhyl-sulfinylphényle spécial, leurs procédés de préparation, les compositions pharmaceutiques les contenant et leur utilisation comme médicaments.

On connaît de EP-B 484223, EP-A 533573, EP-A 551230, EP-A 606175 et DE-A 2555789 certains composés contenant une structure de 2-cyano-3-hydroxy-propénamide; toutefois, ces composés sont différents en structure et ne présentent pas la même puissance inattendue vis-à-vis des enzymes humains pertinents que les nouveaux composés de la présente invention.

Selon un aspect de l'invention, on fournit des composés de formule (I) : 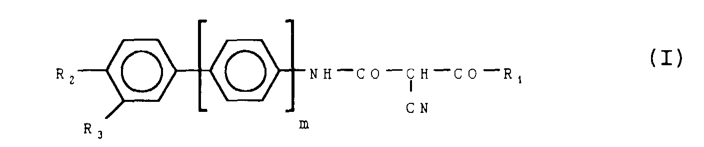 dans laquelle
- R₁: représente un groupement cyclopropyle ou un groupement -CH₂-CH = CH₂;
- R₂: représente un groupement -S(O)ₙ-(CF₂)ₓ-CF₃, dans lequel
- n: désigne 1 ou 2, et x désigne zéro ou 1;
- R₃: représente un atome d'hydrogène ou un radical alkyle contenant de 1 à 3 atomes de carbone; et
- m: désigne zéro ou 1;
ainsi que leurs sels pharmaceutiquement acceptables.

Les composés préférés selon l'invention incluent ceux dans lesquels R₁ représente un groupement cyclopropyle et R₂, R₃ et m sont tels que définis ci-dessus.

D'autres composés préférés selon l'invention incluent ceux dans lesquels R₃ représente un atome d'hydrogène ou un groupement méthyle, de façon tout-à-fait préférentielle un atome d'hydrogène et R₁, R₂ et m sont tels que définis ci-dessus.

Les composés particulièrement préférés selon l'invention sont ceux dans lesquels R₃ représente un atome d'hydrogène ou un groupement méthyle; R₂ représente un groupement -S(O)ₙ-(CF₂)ₓCF₃, R₁ représente un groupement cyclopropyle et m désigne zéro et dans lesquels n désigne 1 ou 2 et x désigne zéro.

Les composés particulièrement préférés selon l'invention sont:
le N-(4'-trifluorométhylsulfinylphényl)2-cyano-3-cyclopropyl-3-hydroxy-prop-2-énamide
le N-(4'-trifluorométhylsulfonylphényl)2-cyano-3-cyclopropyl-3-hydroxy-prop-2-énamide

Les composés de formule (I) sont de nature acide. Les sels d'addition de base des composés de formule (I) peuvent être préparés de manière avantageuse en faisant réagir, dans des proportions approximativement stoechiométriques, une base minérale ou organique avec le composé de formule (I). Les sels peuvent être préparés sans isolement intermédiaire du composé acide correspondant.

Les composés selon l'invention présentent des propriétés pharmacologiques très intéressantes. D'un intérêt particulier est leur activité anti-inflammatoire remarquable. Ils inhibent à la fois la réponse inflammatoire provoquée par des agents irritants, et les réactions d'hypersensibilité retardées en gênant l'activation des cellules immunitaires par un antigène particulier.

L'invention concerne également un procédé de préparation de composés de formule I tel que définis ci-dessus, comprenant la réaction d'un composé de formule (IV) 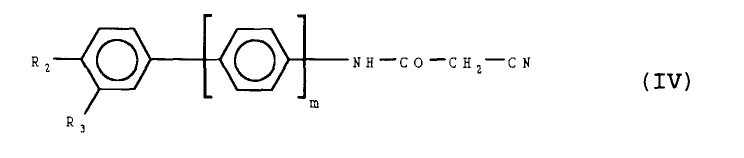 dans laquelle R₂, R₃, m, n et x sont tels que définis ci-dessus, successivement avec une base, par ex. l'hydrure de sodium ou le diisopropylamide de lithium, le cas échéant en présence d'un catalyseur tel que l'imidazole, puis avec un composé de formule (V)

X-CO-R₁ (V)

dans laquelle X représente un groupement labile, par ex. un atome d'halogène, et R₁ est tel que défini ci-dessus. Le composé de formule I ainsi obtenu peut alors être isolé et/ou (si on le souhaite) transformé en un sel.

Les composés de formule IV peuvent par exemple être préparés par l'oxydation de l'atome de soufre d'un composé de formule (IVa) 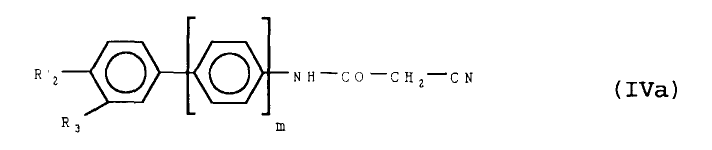 dans laquelle R'₂ désigne un groupement -S-(CF₂)ₓ-CF₃ et R₃, x et m sont tels que définis ci-dessus.

Les composés de formule (IVa) tels que définis ci-dessus peuvent par exemple être préparés par la réaction d'un composé de formule (IIa) 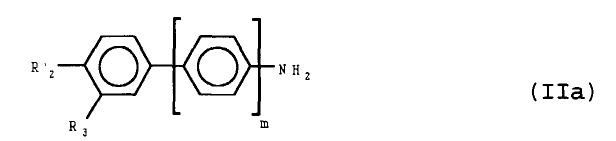 dans laquelle R'₂, x, R₃ et m sont tels que définis ci-dessus, avec un composé de formule III ou un dérivé fonctionnel de celui-ci.

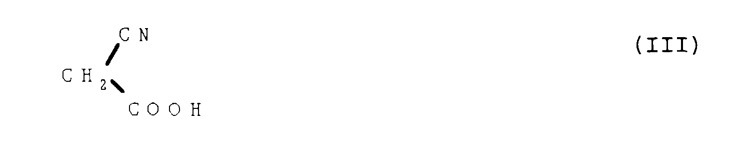

Les composés de formule (IV) peuvent être également préparés en oxydant d'abord l'atome de soufre de composés de formule (IIa) (tel que définis ci-dessus) suivi de la réaction du produit avec un composé de formule (III) (tel que défini ci-dessus) ou un dérivé de celui-ci.

De préférence, la réaction des composés de formule (IIa) et (III) est effectuée en présence de diisopropylcarbodiimide ou de dicyclohexylcarbodiimide dans un solvant organique anhydre tel que le dichlorométhane ou le tétrahydrofuranne. Le dérivé fonctionnel de l'acide de formule (III) est de préférence le chlorure de cyanoacétyle préparé in situ à partir du pentachlorure de phosphore et de l'acide cyanoacétique (voir J. Med. Chem., 1985, 28, 559-568). L'oxydation des composés de formule (IVa) peut être effectuée en utilisant des agents d'oxydation connus tels que par exemple l'acide peracétique. Selon les conditions réactionnelles (par ex. la température, le solvant, le temps de réaction), le groupement trifluorométhylthio peut être oxidé en groupement sulfinyle ou sulfonyle.

La réaction du composé de formule (IV) avec l'hydrure de sodium est de préférence effectuée dans un solvant organique anhydre tel que le tétrahydrofuranne. La réaction avec un composé de formule (V) est également effectuée de préférence dans un solvant organique anhydre tel que le tétrahydrofuranne.

Les composés de formule IV 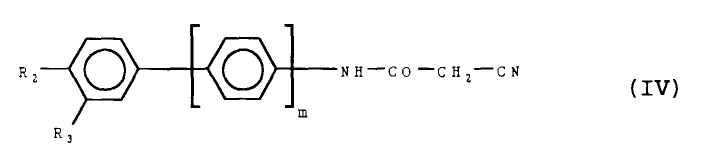 dans laquelle
R₂ représente un groupement : -S(O)ₙ-(CF₂)ₓ-CF₃; dans lequel n désigne 1 ou 2 et x est zéro ou 1;
R₃ représente un atome d'hydrogène ou un radical alkyle contenant de 1 à 3 atomes de carbone; et m désigne zéro ou 1, sont des nouveaux composés et ils constituent une autre disposition de la présente invention.

Les composés de formule (I) et les sels d'addition de base de ceux-ci trouvent une utilisation comme médicaments.

Selon un autre aspect de l'invention, on prévoit l'utilisation, comme médicaments, des composés de formule (I) tels que définis ci-dessus et les sels d'addition de base pharmacologiquement acceptables de ceux-ci.

On préfère utiliser comme médicament les composés selon l'invention dans lesquels R₁ représente un groupement cyclopropyle.

On préfère également utiliser comme médicaments les composés selon l'invention dans lesquels R₃ représente un atome d'hydrogène ou un groupement méthyle, de préférence un atome d'hydrogène.

Les composés particulièrement préférés selon l'invention pour une utilisation comme médicaments sont ceux dans lesquels R₃ représente un atome d'hydrogène ou un groupement méthyle; R₂ représente un groupement -S(O)ₙ-(CF₂)ₓCF₃ et m désigne zéro, n désigne 1 ou 2 et x désigne zéro.

Comme médicaments, on préfère notamment utiliser les composés suivants :
le N-(4'-trifluorométhylsulfinylphényl)2-cyano-3-cyclopropyl-3-hydroxy-prop-2-énamide
N-(4'-trifluorométhylsulfonylphényl)2-cyano-3-cyclopropyl-3-hydroxy-prop-2-énamide.

Les médicaments comprenant un composé de formule I sous forme d'un sel de celui-ci sont utiles, par exemple, dans le traitement de polyarthrite rhumatoïde, des maladies inflammatoires chroniques d'origine immunitaires ou non-immunitaires (par ex. maladie de réaction du greffon contre l'hôte, les réactions de transplantation, uvéite) et du cancer.

Les effets immunosuppresseurs des nouveaux composés peuvent par exemple être mis en évidence par le dosage de l'enzyme dihydro-orotate déshydrogénase.

La dihydro-orotate déshydrogénase (DHO-DH) catalyse la quatrième étape dans la biosynthèse de novo de la pyrimidine. Son inhibition conduirait à l'épuisement des nucléotides pyrimidiniques et par conséquent à une inhibition de la synthèse d'ADN et d'ARN et de la prolifération cellulaire. Les pyrimidines sont également nécessaires pour la glycosylation des lipides et des protéines. La prolifération cellulaire est un composant critique de la réponse immunitaire, au cours de laquelle les cellules immunitaires ont un grand besoin en nucléotides. Un inhibiteur de la DHO-DH serait ainsi un agent immunosuppresseur potentiel et, en outre, aurait des bénéfices thérapeutiques potentiels dans des troubles impliquant une prolifération cellulaire aberrante.

La dose habituelle des nouveaux produits à administrer varie selon le composé utilisé, le patient traité et la maladie en question et peut être par exemple de 0,1 mg à 2000 mg, de préférence de 0,5 à 200 mg par jour par voie orale.

Selon un autre aspect de l'invention, on fournit des compositions pharmacologiques comprenant, à titre de principe actif, au moins un composé de formule (I) tel que défini ci-dessus ou un sel d'addition de base pharmaceutiquement acceptable de celui-ci en association avec un ou plusieurs diluants, supports et/ou excipients pharmacologiquement acceptables.

Pour une utilisation comme médicaments, les composés de formule (I) et leurs sels d'addition de base peuvent par exemple être incorporés dans des compositions pharmaceutiques destinées à l'administration par voie orale, rectale ou parentérale.

Ces compositions pharmaceutiques peuvent par exemple être solides ou liquides et peuvent être sous des formes utilisées de manière classique en médicine humaine, telles que : les comprimés nus ou enrobés, les capsules, les gelules, les granulés, les suppositoires, les solutions, par ex. pour injection; elles peuvent être préparées selon des méthodes classiques.

Le(s) principe(s) actif(s) peut (peuvent) être formulé(s) en compositions pharmaceutiques avec les excipients utilisés de manière classique dans les compositions pharmaceutiques telles que le talc, la gomme arabique, le lactose, l'amidon, le stéarate de magnésium, le beurre de cacao, les véhicules aqueux ou non-aqueux, les matières gras d'origine animale ou végétale, les dérivés paraffiniques, les glycols, divers agent mouillants, dispersants ou émulsionnants et les conservateurs.

Selon un autre aspect de l'invention, on fournit une méthode de traitement de polyarthrite rhumatoïde, de maladies inflammatoires chroniques d'origine immunitaire ou non-immunitaire ou de cancer chez un sujet humain ou animal, comprenant l'administration au sujet d'une quantité efficace d'un composé de formule (I) tel que défini ci-dessus ou un sel d'addition de base pharmacologiquement acceptable de celui-ci.

L'invention est encore illustrée par les exemples non-limitants suivants :

### Exemple 1 : Préparation de N-(4'-trifluorométhylsulfinylphényl)cyanoacétamide et de N-(4'-trifluorométhylsulfonylphényl)cyanoacétamide.

A) Le N-(4'-trifluorométhylthiophényl)cyanoacétamide (4,35 g, 0,0167 mol) (EP 0484223) dans de l'acide acétique (15 ml) a été agité à température ambiante pendant une nuit avec de l'acide peracétique (8,0 ml de 32% p/v, 0,035 mol). Le mélange a été versé sur de la glace/eau et extrait dans de l'acétate d'éthyle. Les extraits ont été lavés par une solution aqueuse de bicarbonate de sodium, séchés (MgSO₄) et évaporés pour donner un solide incolore (4,6 g). La chromotographie sur gel de silice (1-10% d'acétone dans du dichlorométhane) a donné le N-(4'-trifluorométhylsulfinylphényl)cyanoacétamide (2,7 g, 59%) sous forme de cristaux incolores: F 164-165°; IR (KBr) 3280, 1678, 1615, 1592, 1550, 1497, 1405, 1348, 1307, 1262, 1195, 1175, 1141, 1085, 1067 ; ¹H RMN (D₆-DMSO) δ 10,80 (1H, s large), 7,91 (4H, s), 4,03 (2H, s). Ce produit peut être utilisé sans autre purification.
B) Les fractions résiduelles (1,7 g) de la chromatographie précédente ont été réunies et dissoutes dans de l'acide acétique (6 ml) et traitées avec de l'acide peracétique (6 ml). Le mélange a été chauffé à 50°C pendant une nuit, versé dans de l'eau et extrait dans de l'acétate d'éthyle. Les extraits ont été lavés par du bicarbonate de sodium aqueux, séchés (MgSO₄) et évaporés. La chromatographie sur gel de silice (100 g) (2-3% d'acétone dans du dichlorométhane) a donné le N-(4'-trifluorométhylsulfonylphényl)cyanoacétamide sous forme de crystaux incolores (1,06 g, 22%) : F 135-137°; IR (KBr) 3343, 3316, 1724, 1608, 1592, 1538, 1409, 1351, 1217, 1199, 1186, 1170, 1147, 1075 ; ¹H RMN (D₆-DMSO) δ 11,09 (1H, s), 8,15 (2H, d, J = 9), 8,00 (2H, d, J = 9), 4,08 (2H, s). Ce produit peut être utilisé sans autre purification.

### Exemple 2 : Préparation de N-(4'-trifluorométhylsulfinylphényl) 2-cyano-3-cyclopropyl-3-hydroxy-prop-2-énamide

Le N-(4'-trifluorométhylsulfinylphényl)cyanoacétamide (3,1 g, 0,0112 mol) dans du THF sec (100 ml) a été traité avec de l'imidazole (10 mg, catalytique) et l'hydrure de sodium (dispersion d'huile à 80 %) (0,75 g, 0,0246 mol) et agité à température ambiante pendant 90 minutes. Du chlorure de cyclopropylcarbonyle (1,32 ml, 0,0146 mol) a été ajouté goutte à goutte et la solution agitée à température ambiante pendant 10 minutes, versée dans de la glace/HCl 1M et filtrée. Le produit précipité a été récupéré par dispersion, dissous dans du dichlorométhane et passé sur une courte colonne de gel de silice (dichlorométhane) pour donner le N-(4'-trifluorométhylsulfinylphényl)2-cyano-3-cyclopropyl-3-hydroxy-prop-2-énamide (2,54 g, 66%) : F 155-7° ; IR (KBr) 3280, 2219, 1603, 1575, 1536, 1496, 1406, 1353, 1195, 1174, 1141, 1090, 1070, 895 ; ¹H RMN (CDCl₃) δ 15,49 (1H, s), 7,89 (1H, s), 7,82 (4H, s), 2,18 (1H, m), 1,37 (2H, m), 1,22 (2H, m). Analyse: calculé : C₁₄H₁₁F₃N₂O₃S : C, 48,84, H, 3,22 ; N, 8,13 ; F, 16,55 ; S, 9,31.
Trouvé : C, 49,03 ; H, 3,29 ; N, 8,17 ; F, 16,39 ; S, 9,35.

### Exemple 3 : Préparation de N-(4'-trifluorométhylsulfonylphényl)-2-cyano-3-cyclopropyl-3-hydroxy-prop-2-énamide

Le traitement de N-(4'-trifluorométhylsulfonylphényl)cyanoacétamide par la méthode ci-dessus a donné le N-(4'-trifluorométhylsulfinylphényl)2-cyano-3-cyclopropyl-3-hydroxy-prop-2-énamide (65%) : F 168-170° ; IR (KBr) 3300, 2218, 1638, 1579, 1529, 1499, 1411, 1363, 1349, 1322, 1268, 1247, 1216, 1190, 1177, 1142, 1079, 986, 894, 841 ; ¹H RMN (CDCl₃) δ 15,30 (1H, s), 8,03 (2H, d, J = 8,8), 7,92 (1H, s), 7,85 (2H, d, J = 8,8), 2,18 (1H, m), 1,41 (2H, m), 1,25 (2H, m). Analyse: calculé : C₁₄H₁₁F₃N₂O₄S : C, 46,67 ; H, 3,08 ; F, 15,82 ; N, 7,77 ; S, 8,90. Trouvé : C, 47,01 ; H, 3,17 ; F, 15,57, N, 7,84 ; S, 8,79.

### Exemple 4 : Dosage de la dihydro-orotate déshydrogénase (DHO-DH)

La transformation de dihydro-orotate en orotate catalysée par la DHO-DH de la membrane de rate humaine est réalisée par la réduction de la coenzyme Q₁₀. La réoxydation de la coenzyme Q₁₀ est liée à la réduction du dichlorophénolindophénol et elle est suivie par la perte d'absorbance à 650 nm. La vitesse de perte d'absorbance est une mesure directe de la réaction catalysée par l'enzyme DHO-DH. Les vitesses de réaction ont été mesurées en présence d'une gamme de concentrations du composé à tester et on a déterminé à l'aide des graphiques la concentration donnant 50% d'inhibition (CI₅₀) (voir Williamson et al. (1995), "Dihydroorotate dehydrogenase, a high affinity binding protein for A77 1726 and mediator of a range of the biological effects of the immunomodulatory compounds" [La dihydro-orotate déshydrogénase, une protéine de liaison à haute affinité pour A77 1726 et médiatrice d'une gamme d'effets biologiques des composés immunomodulateurs], The Journal of Biological Chemistry).

### Préparation de membranes de rate humaine

Des préparations de membranes brutes ont été réalisées en utilisant une modification d'un protocole de centrifugation différentielle publié. Tous les protocoles ont été effectués dans de la glace ou à une température de 4°C en utilisant des appareils et des tampons pré-refroidis.

Le tissu splénique a été découpé, puis homogénéisé avec un homogénéisateur en verre à téflon (environ 10 passages à 250 t/min) dans 10 volumes de tampons d'homogénéisation (10 mM de Tris-HCl contenant 0,25 M de saccharose, 10 µg/ml d'inhibiteur de trypsine de soja, 2 µg/ml d'aprotinine, de la pepstatine A et de la leupeptine pH 7,4. L'homogénat a été centrifugé à ≈470 g pendant 10 minutes et le surnageant préservé. Les culots ont été rémis en suspension dans 4-6 volumes de tampon d'homogénéisation en utilisant 5 passages de l'homogénéisateur en verre à téflon (250 t/min) et centrifugés pendant 10 minutes à ≈470 g. Le surnageant a été réuni avec celui provenant de la première étape de centrifugation (fraction post-nucléaire); les culots contenant les noyaux et les débris des tissus ont été jetés. Le surnageant post-nucléaire a été centrifugé à 125000 g pendant 40 minutes. Les culots ont été remis en suspension dans du tampon d'homogénéisation (2 ml/g de tissu humide) en utilisant environ 10 passages de l'homogénéisateur en verre à téflon. Des aliquotes de la préparation ont été conservés à -80°C. Les membranes n'ont pas été récongélées une fois décongélées (voir Ozols J. "Preparation of Membrane Fractions" [Préparation de Fractions Membranaires], dans : Methods in Enzymology Volume 182, Guide to Protein Purification (Ed. Deutscher MP), pp 225-235. Academic Press, London, 1990).

L'inhibition de l'activité de la dihydro-orotate déshydrogénase de la rate humaine par cinq composés à tester a été évaluée en utilisant un dosage lié au DCIP (voir Lakaschus, G., and Loffler, M. (1992) Biochem. Pharmacol. 43, 1025-1030). Des membranes (0,33-0,5 mg de protéine) ont été incubées avec 100 µM de coenzyme Q₁₀ dans 50 mM de Tris-HCl, 0,1% de Triton X-100, 1 mM de KCN, pH 8,0. Après une pré-incubation de 90 minutes à 37°C, la réaction a été initiée par l'addition de 500 µM de dihydro-orotate et la réduction de DCIP (200 µM) a été suivie par la perte d'absorbance à 650 nm en utilisant un lecteur de plaque à 96 puits, à 37°C. Les concentrations de médicaments ont été augmentées à des intervalles d'une demi-unité log ou d'une unité log entière, chaque concentration étant testée au moins en triple.

### Résultats

Les résultats suivants ont été trouvés pour trois composés de l'art intérieur (A, B, C) et pour les composés des exemples 3 et 2 dans le test de l'inhibition de l'enzyme DHO-DH humaine :

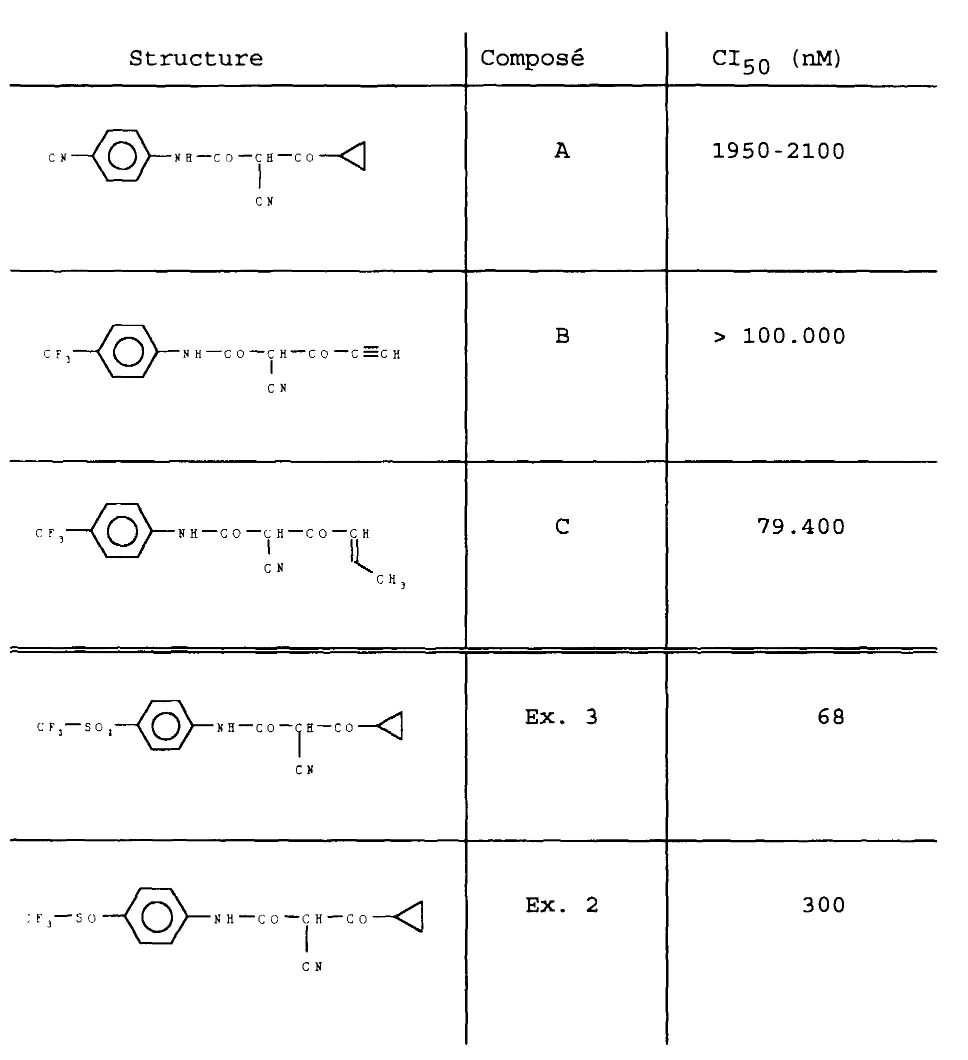

## Revendications

1. Composés de formule (I) : 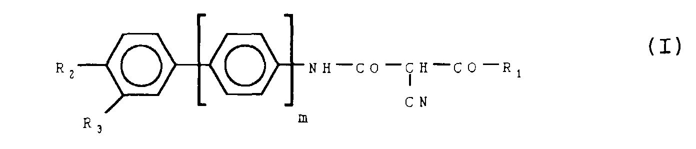 dans laquelle
R₁ représente un groupement cyclopropyle ou un groupement -CH₂-CH = CH₂;
R₂ représente un groupement -S(O)ₙ-(CF₂)ₓ-CF₃, dans lequel
n désigne 1 ou 2, x désigne zéro ou 1;
R₃ représente un atome d'hydrogène ou un radical alkyle contenant de 1 à 3 atomes de carbone;
m désigne zéro ou 1;
ainsi que leurs sels pharmaceutiquement acceptables.

2. Composés selon la revendication 1, caractérisés en ce que R₁ représente un groupement cyclopropyle et R₂, R₃ et m sont tels que définis dans la revendication 1.

3. Composés selon la revendication 1 ou la revendication 2, caractérisés en ce que R₃ représente un atome d'hydrogène ou un groupement méthyle et R₁, R₂ et m sont tels que définis dans la revendication 1 ou la revendication 2.

4. Composé selon l'une quelconque des revendications 1 à 3, choisi parmi :
le N-(4'-trifluorométhylsulfinylphényl)2-cyano-3-cyclopropyl-3-hydroxy-prop-2-énamide et
le N-(4'-trifluorométhylsulfonylphényl)2-cyano-3-cyclopropyl-3-hydroxy-prop-2-énamide et leurs sels pharmaceutiquement acceptables.

5. Procédé de préparation d'un composé de formule I tel que défini dans la revendication 1, comprenant la réaction d'un composé de formule (IV) 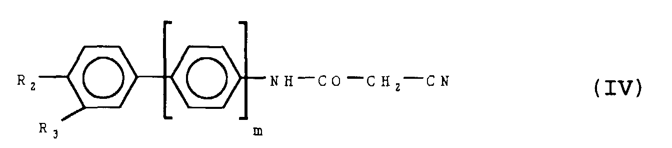 dans laquelle R₂, R₃, m, n et x sont tels que définis ci-dessus, successivement avec une base, le cas échéant en présence d'un catalyseur tel que l'imidazole, puis avec un composé de formule (V)
X-CO-R₁ (V)
dans laquelle X représente un groupement labile, par ex. un atome d'halogène, et R₁ est tel que défini ci-dessus.

6. Procédé selon la revendication 5, caractérisé en ce que les composés de formule 4 tels que définis dans la revendication 5 sont préparés par l'oxydation au niveau de l'atome de soufre d'un composé de formule (IVa) 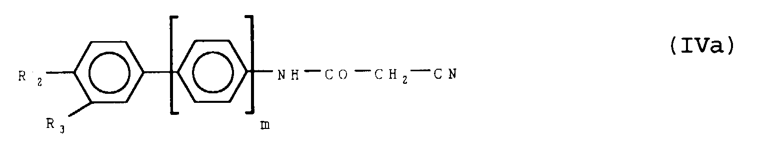 dans laquelle R'₂ désigne un groupement -S-(CF₂)ₓ-CF₃ et R₃, x et m sont tels que définis dans la revendication 1.

7. Procédé selon la revendication 6, caractérisé en ce que les composés de formule (IVa) tels que définis dans la revendication 6 sont préparés par la réaction d'une composé de formule (IIa) 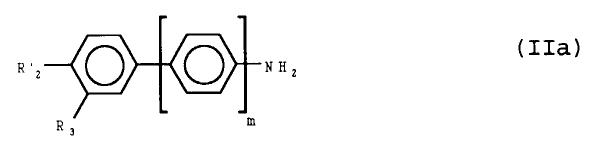 dans laquelle R'₂, x, R₃ et m sont tels que définis dans la revendication 1, avec un composé de formule III ou un dérivé fonctionnel de celui-ci

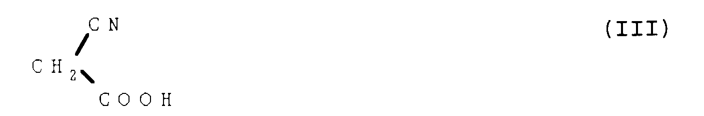

8. Procédé selon la revendication 5, caractérisé en ce que les composés de formule (IV) tels que définis dans la revendication 5 sont préparés par l'oxydation de l'atome de soufre de composés de formule (IIa) tels que définis dans la revendication 7, puis la réaction du produit avec un composé de formule (III) tel que défini dans la revendication 7 ou un dérivé fonctionnel de celui-ci.

9. Composés de formule IV 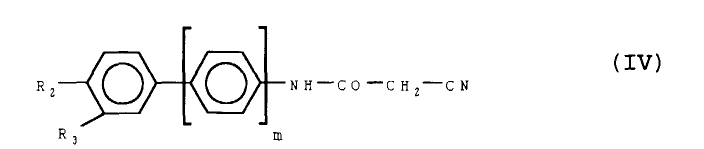 dans laquelle
R₂ représente un groupement : -S(O)ₙ-(CF₂)ₓ-CF₃ ; dans lequel n désigne 1 ou 2 et x est zéro ou 1;
R₃ représente un atome d'hydrogène ou un radical alkyle contenant de 1 à 3 atomes de carbone; et m désigne zéro ou 1.

10. Composition pharmacologique comprenant, à titre de principe actif, au moins un composé de formule (I) tel que défini dans la revendication 1 ou un sel d'addition de base pharmaceutiquement acceptable de celui-ci en association avec un ou plusieurs diluants, supports et/ou excipients pharmacologiquement acceptables.

11. Composé de formule (I) tel que défini dans la revendication 1 ou un sel d'addition de base pharmacologiquement acceptable de celui-ci destiné à une utilisation comme médicament.

12. Composé de formule (I) tel que défini dans la revendication 1 ou un sel d'addition de base pharmacologiquement acceptable de celui-ci destiné à une utilisation dans le traitement de polyarthrite rhumatoide, de maladies inflammatoires chroniques d'origine immunitaire ou non-immunitaire ou de cancer.

## Patentansprüche

1. Verbindungen der Formel (I) 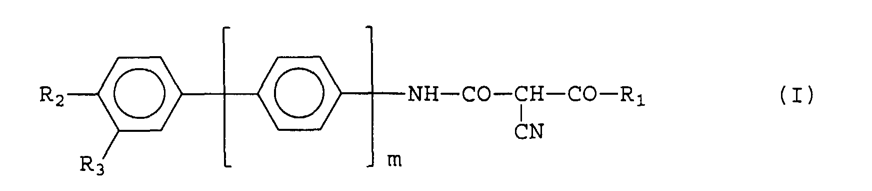 in der
R₁ eine Gruppe Cyclopropyl oder eine Gruppe -CH₂-CH=CH₂ darstellt;
R₂ eine Gruppe -S(O)ₙ-(CF₂)ₓ-CF₃ bedeutet, worin n 1 oder 2 und x null oder 1 bezeichnen;
R₃ ein Wasserstoffatom oder einen Rest Alkyl mit 1 bis 3 Kohlenstoffatomen darstellt;
m null oder 1 bezeichnet;
sowie ihre pharmazeutisch akzeptablen Salze.

2. Verbindungen nach Anspruch 1, dadurch gekennzeichnet, daß R₁ eine Gruppe Cyclopropyl darstellt und R₂, R₃ und m wie in Anspruch 1 definiert sind.

3. Verbindungen nach Anspruch 1 oder Anspruch 2, dadurch gekennzeichnet, daß R₃ ein Wasserstoffatom oder eine Gruppe Methyl darstellt und R₁, R₂ und m wie in Anspruch 1 oder wie in Anspruch 2 definiert sind.

4. Verbindungen nach irgendeinem der Ansprüche 1 bis 3, ausgewählt unter:
N-(4'-Trifluormethylsulfinylphenyl)-2-cyano-3-cyclopropyl-3-hydroxy-prop-2-enamid und
N-(4'-Trifluormethylsulfonylphenyl)-2-cyano-3-cyclopropyl-3-hydroxy-prop-2-enamid und ihren pharmazeutisch akzeptablen Salzen.

5. Verfahren zur Herstellung einer Verbindung der Formel (I) wie in Anspruch 1 definiert, umfassend die Reaktion einer Verbindung der Formel (IV) 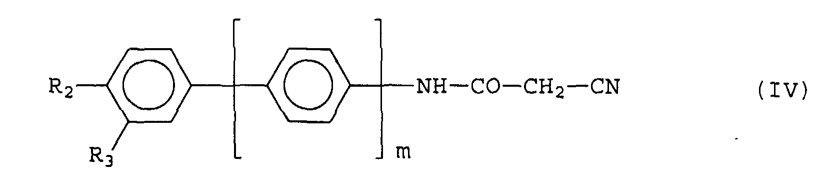 in der
R₂, R₃, m, n und x wie oben definiert sind, mit nacheinander einer Base, gegebenenfalls in Anwesenheit eines Katalysator wie Imidazol, und anschließend mit einer Verbindung der Formel (V)
X-CO-R₁ (V)
in der X eine labile Gruppe ist, beispielsweise ein Halogenatom, und R₁ wie oben definiert ist.

6. Verfahren nach Anspruch 5, dadurch gekennzeichnet, daß die wie in Anspruch 5 definierten Verbindungen der Formel (IV) durch Oxidation im Bereich des Schwefelatoms von einer Verbindung der Formel (IVa) 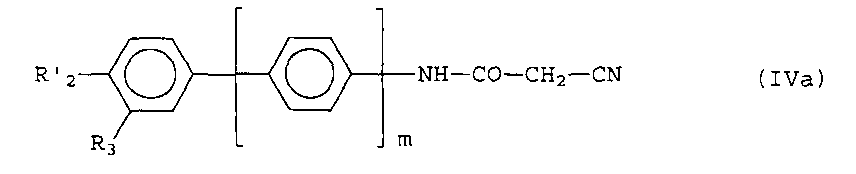 hergestellt werden, in der R'₂ eine Gruppe -S-(CF₂)ₓ-CF₃ bezeichnet und R₃, x und m wie in Anspruch 1 definiert sind.

7. Verfahren nach Anspruch 6, dadurch gekennzeichnet, daß die wie in Anspruch 6 definierten Verbindungen der Formel (IVa) durch Reaktion einer Verbindung der Formel (IIa) 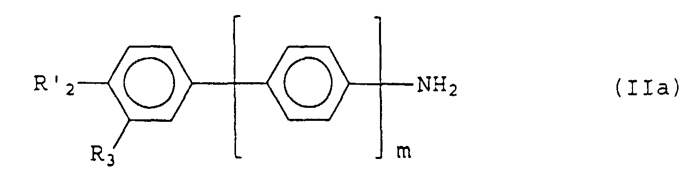 in der R'₂, x, R₃ und m wie in Anspruch 1 definiert sind, mit einer Verbindung der Formel (III) 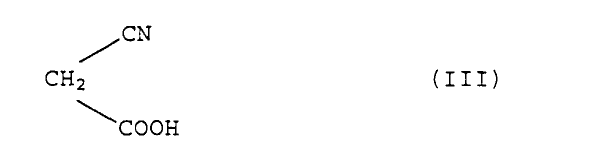 oder einem funktionellem Derivat von ihr hergestellt werden.

8. Verfahren nach Anspruch 5, dadurch gekennzeichnet, daß die wie in Anspruch 5 definierten Verbindungen der Formel (IV) durch Oxidation des Schwefelatoms von wie in Anspruch 7 definierten Verbindungen der Formel (IIa), und anschließende Reaktion des Produktes mit einer wie in Anspruch 7 definierten Verbindung der Formel (III) oder einem funktionellem Derivat von ihr hergestellt werden.

9. Verbindung der Formel (IV) 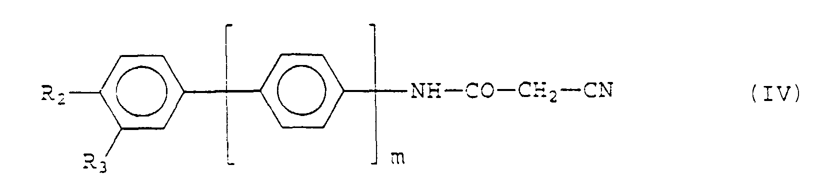 in der
R₂ eine Gruppe -S(O)ₙ-(CF₂)ₓ-CF₃ darstellt, worin n 1 oder 2 und x null oder 1 bezeichnen;
R₃ ein Wasserstoffatom oder einen Rest Alkyl mit 1 bis 3 Kohlenstoffatomen darstellt und m null oder 1 bezeichnet.

10. Pharmakologische Zusammensetzung, enthaltend als Wirkstoff mindestens eine wie in Anspruch 1 definierte Verbindung der Formel (I) oder ein Additionssalz von ihr mit pharmazeutisch akzeptablen Basen in Assoziation mit einem oder mehreren, pharmakologisch akzeptablen Verdünnungsmitteln, Trägerstoffen und/oder Füllstoffen.

11. Verbindung der Formel (I) wie in Anspruch 1 definiert oder ein pharmakologisch akzeptables Baseadditionssalz von ihr, vorgesehen für eine Verwendung als Arzneimittel.

12. Verbindung der Formel (I) wie in Anspruch 1 definiert oder ein pharmakologisch akzeptables Baseadditionssalz von ihr, vorgesehen für eine Verwendung zur Behandlung von rheumatoider Arthritis, chronischen entzündlichen Erkrankungen immunitären oder nicht immunitären Ursprungs oder von Krebs.

## Claims

1. Compounds of formula (I) : 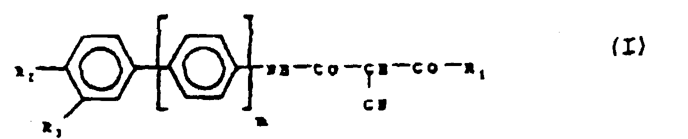 in which
R₁ represents a cyclopropyl group or a -CH₂-CH = CH₂ group;
R₂ represents a -S(O)ₙ-(CF₂)ₓ-CF₃ group, in which
n designates 1 or 2, x designates zero or 1;
R₃ represents a hydrogen atom or an alkyl radical containing 1 to 3 carbon atoms;
m designates zero or 1;
as well as their pharmaceutically acceptable salts.

2. Compounds according to claim 1, characterized in that R₁ represents a cyclopropyl group, and R₂, R₃ and m are as defined in claim 1.

3. Compounds according to claim 1 or claim 2, characterized in that R₃ represents a hydrogen atom or a methyl group and R₁, R₂ and m are as defined in claim 1 or claim 2.

4. Compound according to any one of claims 1 to 3, chosen from:
N-(4'-trifluoromethylsulphinylphenyl)2-cyano-3-cyclopropyl-3-hydroxy-prop-2-enamide and
N-(4'-trifluoromethylsulphonylphenyl)2-cyano-3-cyclopropyl-3-hydroxy-prop-2-enamide and their pharmaceutically acceptable salts.

5. Preparation process for a compound of formula I as defined in claim 1, comprising the reaction of a compound of formula (IV) 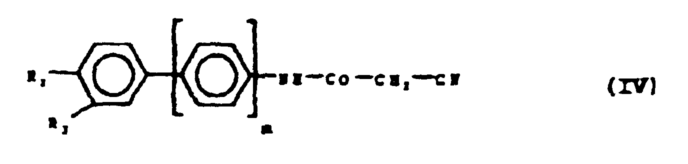 in which R₂, R₃, m, n and x are as defined above, successively with a base, if appropriate in the presence of a catalyst such as imidazole, then with a compound of formula (V)
X-CO-R₁ (V)
in which X represents a labile group, for example a halogen atom, and R₁ is as defined above.

6. Process according to claim 5, characterized in that the compounds of formula 4 as defined in claim 5 are prepared by oxidation at the level of the sulphur atom of a compound of formula (IVa) 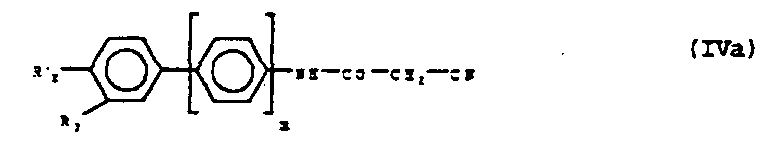 in which R'₂ designates an -S-(CF₂)ₓ-CF₃ group and R₃, x and m are as defined in claim 1.

7. Process according to claim 6, characterized in that the compounds of formula (IVa) as defined in claim 6 are prepared by reacting a compound of formula (IIa) 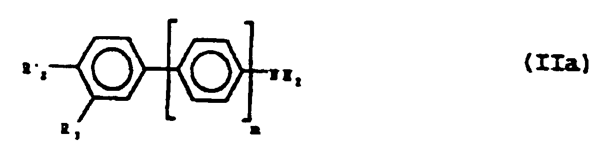 in which R'₂, x, R₃ and m are as defined in claim 1, with a compound of formula III or a functional derivative of this

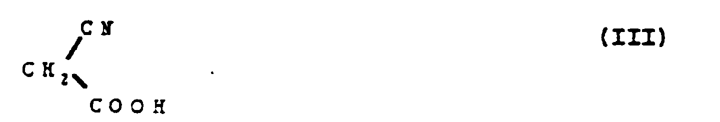

8. Process according to claim 5, characterized in that the compounds of formula (IV) as defined in claim 5 are prepared by the oxidation of the sulphur atom of compounds of formula (IIa) as defined in claim 7, then reacting the product with a compound of formula (III) as defined in claim 7 or a functional derivative of this.

9. Compounds of formula IV 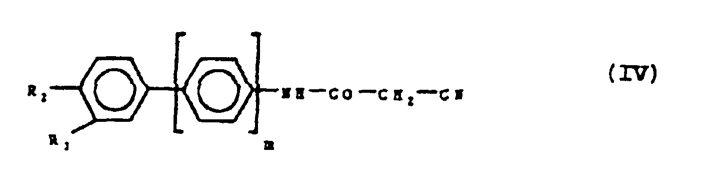 in which
R₂ represents an -S(O)ₙ-(CF₂)ₓ-CF₃ group in which n designates 1 or 2 and x is zero or 1;
R₃ represents a hydrogen atom or an alkyl radical containing 1 to 3 carbon atoms; and m designates zero or 1.

10. Pharmacological composition comprising at least one compound of formula (I) as defined in claim 1 or a pharmaceutically acceptable base addition salt of this combined with one or more pharmacologically acceptable diluents, supports and/or excipients as an active ingredient.

11. Compound of formula (I) as defined in claim 1 or a pharmacologically acceptable base addition salt of this intended to be used as a medicament.

12. Compound of formula (I) as defined in claim 1 or a pharmacologically acceptable base addition salt of this intended to be used in the treatment of rheumatoid polyarthritis, chronic inflammatory diseases of immunological or non-immunological origin, or cancer.
